# EUROPÄISCHE PATENTSCHRIFT

(11) **EP 2 980 070 B1**
(45) Veröffentlichungstag und Bekanntmachung des Hinweises auf die Patenterteilung: **20.06.2018**
(21) Anmeldenummer: 15177200.1
(22) Anmeldetag: 17.07.2015
(51) Int. Cl.: B01J 23/38, C07C 29/52, C07D 225/02, C07C 45/00, C07C 45/51, C07D 201/04, C07C 45/58, B01J 23/44, C07C 45/82, C07D 201/06, C07C 45/28, C07D 301/12, C08G 69/16, C07C 249/04

(54) **VERFAHREN ZUR HERSTELLUNG VON CYCLODODECANON**
METHOD FOR PREPARATION OF CYCLODODECANONE
PROCÉDÉ DESTINÉ À LA FABRICATION DE CYCLODODÉCANONE

(30) Priorität: 01.08.2014 EP 14179449
(43) Veröffentlichungstag der Anmeldung: 03.02.2016
(73) Patentinhaber: Evonik Degussa GmbH, 45128 Essen (DE)
(72) Erfinder: MICOINE, Kévin, 45701 Herten (DE); MEIER, Ralf, 44265 Dortmund (DE); HERWIG, Jürgen, 46569 Hünxe (DE); ROOS, Martin, 45721 Haltern am See (DE); HÄGER, Harald, 59348 Lüdinghausen (DE); CAMERETTI, Luca, 44267 Dortmund (DE); DÖRING, Jens, 44141 Dortmund (DE)

(56) Entgegenhaltungen:
- EP-A1- 1 090 900
- EP-A1- 1 329 448
- EP-A1- 1 411 050
- Thomas Schiffer, Georg Oenbring: "Cyclododecanol, Cyclododecanone, and Laurolactam" In: "Ullmann's Encyclopedia of Industrial Chemistry", 2005, Wiley, XP002734395, Bd. 11, Seiten 1-5, DOI: 10.1002/14356007.a08_201, * Seite 1 Reaktionschema, Seite 4, linke Spalte, vierter Absatz von oben, Seite 4, rechte Spalte, letzter Absatz;; Abbildung 1 *

## Beschreibung

Die vorliegende Erfindung betrifft ein Verfahren zur Herstellung vor Cyclododecanon, ein Verfahren zur Herstellung von Laurinlactam sowie ein Verfahren zur Herstellung von Polyamid 12.

Cyclododecanon (CDON) wird für die Synthese von Laurinlactam eingesetzt. Das Lactam wiederum eignet sich für die Herstellung von Polyamid 12.

Die Herstellung von CDON kann ausgehend von Cyclododecatrien (CDT) erfolgen. Zunächst kann eine Selektivhydrierung von Cyclododecatrien (CDT) in Cyclododecen (CDEN) vorgenommen werden. Anschließend folgen eine Epoxidierung von CDEN zu Epoxycyclododecan (CDANepoxid) und die Umlagerung von CDANepoxid zu Cyclododecanon (CDON). Ausgehend von CDEN umfasst die CDON-Synthese folgende Schritte:
a. Epoxidierung von Cyclododecen (CDEN) zu Epoxycyclododecan (CDANepoxid) und
b. Umlagerung des CDANepoxids zu CDON unter Erhalt eines Gemisches, das Cyclododecan (CDAN) enthält.

Das Gemisch (CDON-haltiges Gemisch), das aus der Umlagerung erhalten wird, umfasst somit zumindest CDON und CDAN.

Bei diesem Herstellungsverfahren von CDON tritt das Problem auf, dass signifikante Mengen an CDAN oder CDEN entstehen können. Dieses Problem wird mit der Alterung von Katalysatoren, die gegebenenfalls eingesetzt werden, verschärft. Die anfallende Menge an Nebenprodukten kann mehr als 20 Gew.-%, bezogen auf das resultierende, CDON-haltige Gemisch aus der Umlagerung, betragen.

Der hohe Anteil an CDAN kann sich für die nachfolgenden Reaktionen wie die Herstellung von Laurinlactam nachteilig auswirken.

Insofern bestand die Aufgabe der vorliegenden Erfindung darin, ein neues Verfahren zur Herstellung von CDON zur Verfügung zu stellen, durch das der Anteil an CDAN im Endprodukt CDON verringert wird. Zudem sollte das Herstellungsverfahren insgesamt wirtschaftlicher erscheinen. EP 1 090 900 A1 offenbart ein Verfahren zur Herstellung von Cyclododecanon (CDON) ausgehend von Cyclododecan-epoxid (CDANepoxid). Demgemäß wurde ein neues Verfahren zur Herstellung von CDON umfassend die eingangs genannten Schritte a und b gefunden, welches im Folgenden als Reaktionsroute I bezeichnet wird. Das CDAN wird aus dem CDON-haltigen Gemisch abgetrennt und zu CDON oxidiert (erfindungsgemäßes CDON-Verfahren).

Das erfindungsgemäße CDON-Verfahren kann kontinuierlich oder diskontinuierlich durchgeführt werden.

Die Umlagerung wird vorzugsweise bei einem maximalen Wasserstoffdruck von 0,9 bar durchgeführt. Bevorzugt beträgt der Wasserstoffdruck 0 bis 0,9 bar, besonders bevorzugt 0 bis 0,5 bar. Das erfindungsgemäße Verfahren kann ohne Wasserstoff durchgeführt werden, es ist jedoch zur Unterbindung ungesättigter Nebenprodukte bevorzugt, zumindest einen geringen Wasserstoffanteil vorzulegen. Dieser kann 0,05 bis 0,5 bar, vorzugsweise 0,1 bis 0,4 bar aufweisen.

Die zuvor genannten Druckangaben beziehen sich auf den Partialdruck an Wasserstoff in dem System. Üblicherweise sind Komponenten des Reaktionsgemisches, einschließlich des Lösemittels, Luft oder Inertgase wie Stickstoff oder Argon weitere gasförmige Bestandteile des Systems.

Als eine Umlagerung im Sinne des erfindungsgemäßen CDON-Verfahrens wird insbesondere eine Reaktion verstanden, bei der mindestens 90 Gew.-% CDON, bezogen auf das Gesamtgewicht an entstehendem CDON und CDOL, erhalten wird.

Vorzugsweise wird das CDEN für die Epoxidierung (Schritt a) aus CDT erhalten. Das CDT wiederum kann aus 1,3-Butadien gewonnen werden. Die Selektivhydrierung von CDT kann in der Gasphase mit einem niedrigen Partialdruck an Wasserstoff und einem Pd-haltigen Katalysator erfolgen (EP 1457476). Aus dem Schritt entstehen üblicherweise 5 bis 15 Gew.-% CDAN (Cyclododecan) in CDEN.

Die Epoxidierung von CDEN gemäß Schritt a kann mit Wasserstoffperoxid, mittels eines Phasentransferkatalysators und eines Metallsalzes im sauren pH durchgeführt werden. Das im CDEN enthaltende CDAN wirkt als Absetzbeschleuniger (EP 1411050, EP 1411051). Nach der Reaktion wird CDANepoxid erhalten, das weiterhin CDAN, unreagiertes CDEN oder beides umfassen kann.

CDAN kann vor der Umlagerung (Schritt b) zumindest teilweise abgetrennt werden. Vorzugsweise wird dieses CDAN zu CDON oxidiert.

Bei der Oxidation von CDAN zu CDON kann zumindest teilweise CDOL entstehen. Das CDOL kann danach zu CDON dehydriert werden.

Die Umlagerung gemäß Schritt b erfolgt vorzugsweise in Gegenwart eines edelmetallhaltigen Katalysators (Katalysatorsystem), wobei der Katalysator bevorzugt Titandioxid, Zirconiumdioxid oder beides enthält. In diesem Reaktionsschritt bildet sich CDON, das CDAN als Nebenprodukt enthält (CDON-haltiges Gemisch). Weiterhin können CDEN, CDOL oder Mischungen daraus als Nebenprodukte enthalten sein. Ebenso können weitere Nebenprodukte enthalten sein, die einen gegenüber CDON höheren Siedepunkt aufweisen (Hochsieder). Vorzugsweise wird die Umlagerung nicht in Gegenwart von Alkalimetallhydroxiden als Katalysator durchgeführt.

Das Edelmetall des Katalysatorsystems wird vorzugsweise ausgewählt aus Ruthenium, Rhodium, Palladium, Osmium, Iridium und Platin, wobei Ruthenium, Palladium und Platin bevorzugt sind und Palladium besonders bevorzugt ist. Das Edelmetall kann als Pulver (ungeträgert) oder geträgert vorliegen. In Pulverform sind beispielsweise elementare Edelmetalle oder ihre Oxide geeignet.

Weiterhin kann mindestens ein Metalloxid als weiterer Bestandteil des Katalysatorsystems enthalten sein. Das Metalloxid des Katalysatorsystems umfasst Titandioxid, Zirconiumdioxid oder Mischungen daraus oder besteht aus mindestens einem der zuvor genannten Oxide. Hiervon sind auch mit Titandioxid oder Zirconiumdioxid dotierte oder beschichtete Stoffe wie Aluminiumoxid oder Siliciumdioxid umfasst.

Das Metalloxid des Katalysatorsystems kann als Träger für das Edelmetall des Katalysatorsystems fungieren. Das Edelmetall kann wahlweise auf einem alternativen Träger aufgebracht sein, der beispielsweise aus Aluminiumoxid, Siliciumdioxid oder Aktivkohle ausgewählt ist. Titandioxid oder Zirconiumdioxid sind bevorzugte Träger.

Die Metalloxide des Katalysatorsystems sowie die alternativen Träger können als Pulver oder als Formkörper vorliegen. Geeignete Formkörper sind Kugeln, Extrudate, Tabletten, Granulate und Pellets. Es ist bevorzugt, dass die Träger des Edelmetalls als Formkörper vorliegen. Es ist ebenso bevorzugt, dass das Metalloxid des Katalysatorsystems, wenn es nicht als Träger fungiert, als Formkörper vorliegt.

Das Katalysatorsystem kann folglich unabhängig voneinander als eines der folgenden Systemformen vorliegen:
I) Das Edelmetall ist nicht geträgert; als Metalloxid des Katalysatorsystems ist mindestens Titandioxid oder Zirconiumdioxid enthalten;
II) das Edelmetall ist geträgert, wobei der Träger nicht Titandioxid und/oder Zirconiumdioxid enthält oder daraus besteht. Das System enthält zusätzlich zumindest ein Metalloxid, welches ausgewählt ist aus Titandioxid oder Zirconiumdioxid.
III) Das Edelmetall ist auf einem Metalloxid geträgert, welches aus Titandioxid und Zirconiumdioxid ausgewählt ist, wobei vorzugsweise kein Titandioxid enthalten ist.

Die Systemformen II und III sind bevorzugt, wobei Systemform III besonders bevorzugt ist.

Geeignetes Titandioxid als Metalloxid des Katalysatorsystems kann durch das Sulfatverfahren, das Chloridverfahren oder durch Flammhydrolyse (Pyrogenverfahren) von Titantetrachlorid erhalten werden. Sämtliche Verfahren sind dem Fachmann bekannt. Geeignete Modifikationen sind Rutil und Anatas, wobei das eingesetzte Titandioxid Mischungen der genannten Modifikationen enthalten kann.

Das durch das Sulfat- oder Chloridverfahren hergestellte Titandioxid kann in Wasser sauer reagieren, wobei die Verbindungen üblicherweise einen pH-Wert von 3 oder weniger aufweisen (saures Titandioxid). Ebenso enthält saures Titandioxid meist mehr als 5 Gew.-%, bezogen auf das Gesamtgewicht des Titandioxid-Trägers, an Stoffen wie Titanylsulfat oder Titanylhydroxid auf. Ein Titandioxid auf Basis eines sauren Titandioxids ist kommerziell als Aerolyst 7750 erhältlich (Evonik, Deutschland). Saures Titanoxid ist für das vorliegende Verfahren weniger bevorzugt. In anderen Worten ist es bevorzugt, kein saures Titandioxid einzusetzen. Geeignetes, nicht-saures Titandioxid, welche bevorzugt ist, zeigt in Wasser einen pH-Wert von 5 oder mehr.

Besonders bevorzugtes Titandioxid wird mittels Flammpyrolyse gewonnen, wie es beispielsweise in DE-A-830786 beschrieben ist.

Geeignetes Titandioxid ist unter der Bezeichnung Aeroxid P25 Titandioxid (Pulver) oder Aerolyst 7711 (Formkörper) der Firma Evonik, Deutschland, sowie Hombikat M234 (Formkörper) der Firma Sachtleben, Deutschland, erhältlich.

Zirconiumdioxid (Zirconium(IV)-oxid) ist beispielsweise aus Zirconiumhydroxid erhältlich, wobei es bei über 200 °C, beispielsweise bei 350 °C, kalziniert wird. Das Zirconiumdioxid kann beispielsweise mit Yttriumoxid dotiert sein.

Geeignetes Zirconiumdioxid ist monoklin oder tetragonal. Mischungen dieser Modifikationen sind möglich.

Das Metalloxid des Katalysatorsystems kann eine durchschnittliche Schüttdichte von 0,5 bis 2 g/cm³ aufweisen.

Das Metalloxid des Katalysatorsystems kann eine BET-Oberfläche von mindestens 5 m²/g aufweisen.

Der Anteil an Edelmetall, bezogen auf das Gesamtgewicht aus Edelmetall und Träger, kann 0,01 bis 5 Gew.-%, vorzugsweise 0,05 bis 1,2 Gew.-% und bevorzugt 0,1 bis 0,6 Gew.-% betragen.

Das Edelmetall kann auf oder im Träger verteilt sein.

Der Stoffmengenanteil an Edelmetall, bezogen auf die Stoffmenge CDAN-epoxid, kann 0,00001 bis 0,1, vorzugsweise 0,0001 bis 0,01, betragen.

Der Stoffmengenanteil an Metalloxid des Katalysatorsystems, bezogen auf die Stoffmenge der Verbindung E, kann 0,01 bis 100, vorzugsweise 0,01 bis 10, betragen.

Das CDAN wird vom Reaktionsgemisch abgetrennt und zu CDON oxidiert.

Das verbleibende Gemisch umfassend CDON und Hochsieder einschließlich CDOL kann in Gegenwart von Wasserstoff und einem Katalysator hydriert werden, um die ungesättigten Nebenprodukte zu entfernen. Anschließend wird das reine Produkt CDON von einer Hochsiederfraktion einschließlich CDOL beispielsweise per Destillation abgetrennt.

Aus dieser Hochsiederfraktion kann CDOL anschließend abdestilliert werden und CDOL kann innerhalb der Reaktionsroute I mittels eines Dehydrierungskatalysators in CDON umgesetzt werden. Es ist jedoch bevorzugt, das abgetrennte CDOL vor der Dehydrierung einer Reaktionsroute II zur Herstellung von CDON zuzuführen.

Die Reaktionsroute II umfasst die folgenden Schritte:
a. Hydrierung von CDT zu CDAN,
b. Oxidation von CDAN zu einem Gemisch umfassend CDOL und CDON und
c. Dehydrierung von CDOL zu CDON.

Geeignete Katalysatoren für die Dehydrierung von CDOL enthalten Kupfer oder Kupferverbindungen wie beispielsweise Kupfer-(II)-oxid.

Das CDOL aus der Route I wird vorzugsweise vor Schritt c der Route II, der Dehydrierung, eingespeist.

Das aus der Route I abgetrennte CDAN wird vor Durchführung der Oxidation der Reaktionsroute II zugeführt. Das CDAN stammt aus dem CDON-haltigen Gemisch. CDAN, das nach der Epoxidierung abgetrennt wurde, kann mit dem CDAN aus dem CDON-haltigen Gemisch vereinigt werden. Sofern das abgetrennte CDAN aus Route I 0,1 bis 99 Gew.-% CDEN, enthält, bezogen auf das Gesamtgewicht aus CDAN und CDEN, ist es bevorzugt, dieses Gemisch der Hydrierung von CDT gemäß Schritt a der Reaktionsroute II zuzuführen. Hierdurch wird CDEN zu CDAN hydriert, welches im Schritt b der Route II oxidiert werden kann.

Hinsichtlich des CDEN-Anteils gibt es einen Überlappungsbereicht im Bereich von 0,1 bis 0,5 Gew.-%. Hierbei kann der Fachmann auswählen, ob er das CDEN-haltige CDAN dem Schritt a oder dem Schritt b der Route II zuführt.

Insofern können die Reaktionsrouten I und II in der Art kombiniert werden, dass anfallendes CDAN bzw. CDOL aus der Route I abgetrennt und entfernt und in Route II überführt werden. Hierbei ist es bevorzugt, die beiden Routen kontinuierlich durchzuführen. Diese besondere Ausführungsform der Erfindung nutzt Nebenprodukte der Route I zur Weiterverarbeitung in Route II. Dies ist besonders wirtschaftlich und ökologisch. Eine Entsorgung der Nebenprodukte der Route I entfällt.

Die Abtrennung von CDEN, CDAN bzw. CDOL sowie der weiteren Hochsieder kann durch dem Fachmann geläufige Methoden vorgenommen werden. Hierbei ist die Destillation bevorzugt. Besonders bevorzugt erfolgen sämtliche Abtrennungen mittels Destillation. Es ist vorteilhaft, mehrere Destillationen nacheinander durchzuführen (mehrstufige Destillation).

Das CDAN kann nach der Epoxidierung abdestilliert werden.

Nach der Umlagerung ist es vorteilhaft, zunächst das CDAN bzw. ein Gemisch aus CDAN und CDEN (Leichtsiederfraktion) abzudestillieren und den Rückstand enthaltend CDON, CDOL und weitere Hochsieder erneut einer Destillation zu unterziehen. Hierbei lässt sich CDON gewinnen, das von einer Hochsiederfraktion enthaltend CDOL abgetrennt wird. CDOL wiederum kann von den verbleibenden Hochsieder-Produkten abdestilliert werden.

Leichtsieder sind Stoffe mit einem Siedepunkt, der geringer ist als der Siedepunkt von CDON.

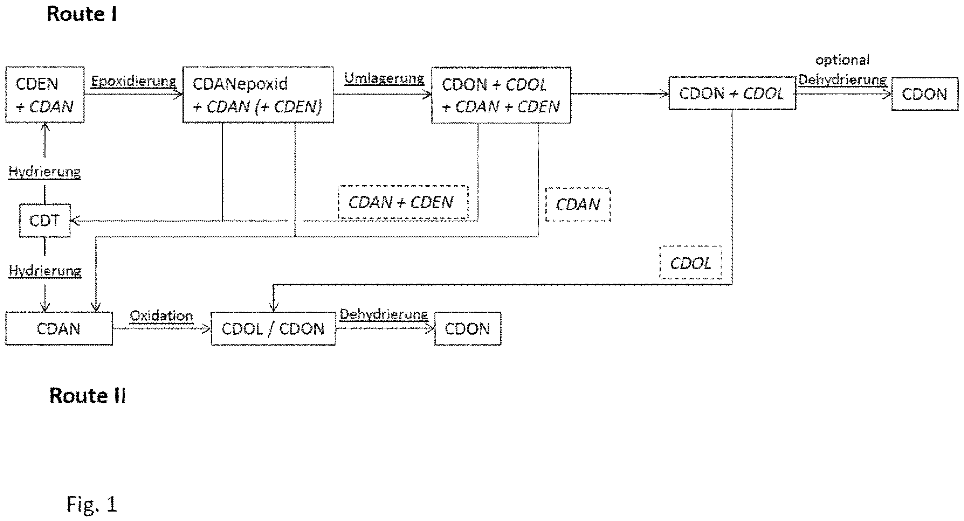

Die Fig. 1 stellt den Stoffverlauf mit den entsprechenden Reaktionen dar. Die kursiv darstellten Verbindungen sind Nebenprodukte der jeweiligen Reaktion.

Ausgehend von CDT wird via Route I mittels Hydrierung (Selektivhydrierung) CDEN erhalten, das zu CDANepoxid epoxidiert wird. Anschließend erfolgt eine Umlagerung zu CDON, wobei vorhandenes CDAN - beispielsweise mittels Destillation - abgetrennt wird. Das nach der Epoxidierung enthaltende CDAN kann ebenfalls abgetrennt werden. Das abgetrennte CDAN kann CDEN enthalten. Vorzugsweise wird das CDAN jedoch der Umlagerung zugeführt und erst nach der Umlagerung abgetrennt. Das abgetrennte CDAN wird bevorzugt in die Route II überführt, wobei die einzelnen Fraktionen aus Route II bevorzugt zusammengeführt werden.

Das verbleibende CDON kann CDOL enthalten, das abgetrennt und der Route II zugeführt werden kann. Alternativ kann das CDOL innerhalb der Route I zu CDON dehydriert werden.

Ein weiterer Gegenstand der Erfindung ist ein Verfahren zur Synthese von Laurinlactam (erfindungsgemäßes Lactamverfahren), bei dem das zuvor genannte erfindungsgemäße Verfahren zur Herstellung von CDON herangezogen wird. Hierbei wird das Laurinlactam aus CDON erhalten, wobei das CDON gemäß dem erfindungsgemäßen CDON-Verfahren hergestellt wird.

Ein weiterer Gegenstand der Erfindung ist ein Verfahren zur Synthese von Polyamid 12 (erfindungsgemäßes Polyamidverfahren), bei dem das zuvor genannte erfindungsgemäße Verfahren zur Herstellung von CDON herangezogen wird.

Das im erfindungsgemäßen CDON-Verfahren produzierte CDON kann im erfindungsgemäßen Lactam- oder Polyamidverfahren unter Erhalt von Cyclododecanon-oxim (CDON-oxim) oximiert werden. Im Folgeschritt kann die Beckmann-Umlagerung zum Laurinlactam erfolgen, wobei die Umlagerung mittels Schwefelsäure oder Cyanurchlorid erfolgen kann. Das Lactam kann unter Polykondensation zu Polyamid weiterverarbeitet werden.

Die Oximierung, die Beckmann-Umlagerung sowie die Kondensationsreaktion sind dem Fachmann bekannt.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von einem Beispiel näher erläutert. Alternative Ausführungsformen der vorliegenden Erfindung sind in analoger Weise erhältlich.

### Beispiel 1

### Epoxidroute = Reaktionsroute I

### konventionelle Route = Reaktionsroute II

### Selektivhydrierung von CDT:

1000 kg/h CDT wurden in einer Sättigungskolonne als Gas-Flüssigkontaktapparat bei einem Druck von 0,75 bar und einer Temperatur von 155°C kontinuierlich in einen 10 m³/h Kreisgasstrom, der im wesentlich aus Stickstoff bestand, verdampft. Der sich ergebende Gasstrom wurde einem Festbettreaktor mit einem Pd auf Al₂O₃ Katalysator zugeführt, in dem bei einer Temperatur von 130 °C die Reaktion mit Wasserstoff erfolgte. Dazu wurden 25 kg/h Wasserstoff am Eingang des Reaktors zum Kreisgasstrom dosiert. Am Ausgang des Reaktors wurde aus dem Kreisgasstrom das Produkt bei 35 °C auskondensiert. Die auskondensierte Flüssigkeit hatte eine Zusammensetzung von 84,8 % CDEN und 15 % CDAN. CDDIEN- und CDT-Isomere liegen in Konzentrationen kleiner 0,2 % vor.

Der verbleibende Kreisgasstrom wurde über einen Verdichter wiederum der Sättigungskolonne zugeführt (siehe oben).

### Epoxidierung von CDEN:

Die Mischung von CDEN (84,8%) und CDAN (15 %) aus der Selektivhydrierung wurde in einer dreistufigen Reaktorkaskade mit H₂O₂ epoxidiert.

Die CDAN/CDEN Mischung wurde zuerst in einen ersten 8 L-Reaktor kontinuierlich mit einer Dosierrate von 2 kg/h zudosiert. Es wurde zusätzlich eine 50%ige H₂O₂ Lösung (544 g/h), eine wässrige Lösung von 30 Gew-% Na₂WO₄ und 18 Gew-% H₃PO₄ (125 g/h), eine 50%ige Lösung von Trioctylamin in CDEN (68 g/h) und Wasser (150 g/h) in das 5,5 L zweiphasige Reaktionsgemisch bei einer Temperatur von 80 °C zudosiert.

Die zweiphasige Mischung kam aus dem ersten Reaktor mit einem Umsatz des CDEN von 79% und lief in einen zweiten 8 L-Reaktor über. Dazu wurde eine 50%ige H₂O₂ Lösung (131 g/h) in das 5,5 L zweiphasige Reaktionsgemisch bei einer Temperatur von 78 °C zudosiert.

Aus diesem zweiten Reaktor kam ein zweiphasige Mischung mit einem Umsatz des CDEN von 96% und wurde in einen Reaktor mit einem Füllstand von 25 L geleitet. Das zweiphasige Reaktionsgemisch wurde in diesem Reaktor bei einer Temperatur von 76 °C gerührt.

Die beiden Phasen der Reaktionsmischung aus dem 25 L-Reaktor wurden in einem Phasentrennbehälter getrennt. Die organische Phase wurde anschließend mit einer 5%ige NaOH Lösung (0,5 kg/h) in einem 5 L-Behälter gewaschen. Beide Phasen wurden anschließend in einem Trennbehälter getrennt.

Die organische Phase wurde einer kontinuierlichen, zweistufigen Destillation zugeführt. In einer ersten Kolonne (Kolonne mit 8 m Gewebepackung; Oberfläche von 500 m²/m³: Kopfdruck 300 mbar) wurde das restliche Wasser als Destillat abgetrennt. Der Sumpf wurde in einer zweiten Kolonne (Kolonne mit 6 m Gewebepackung; Oberfläche von 500 m²/m³; Kopfdruck 10 mbar) destilliert und das gewünschte Produkt mit 85-90% CDAN-Epoxid und 5-10% CDAN wurde mit einer Rate von 2,1 kg/h im Destillat erhalten. Es entspricht eine gesamte Ausbeute von 97% für die Epoxidierung.

### Umlagerung von CDAN-Epoxid in CDON:

Das CDAN-Epoxid aus dem vorherigen Schritt wurde mittels eines 0,5 % Pd/ZrO₂ Katalysators in einer dreistufigen Reaktorkaskade in CDON umgesetzt.

Die Mischung von CDAN-Epoxid (84,8%) und CDAN (15 %) aus der Epoxidierung wurde in einen ersten Kreislaufreaktor mit einer Dosierrate von 2 kg/h zudosiert. Der Kreislaufreaktor bestand aus einem 12 L-Rohrreaktor, der mit 10 kg 0,5% Pd/ZrO₂ Festbettkatalysator bei einer Temperatur von 200 °C gefüllt war und aus einem 8 L-Vorlagebehälter gespeist wurde.

Aus dem ersten Kreislaufreaktor kam eine Mischung von 59% CDON, 18% CDAN-Epoxid, 16% CDAN, 0,4% CDEN und 1,9% CDOL. Dieses Gemisch wurde in einen zweiten Kreislaufreaktor mit einer Dosierrate von 2 kg/h zudosiert. Dieser zweite Kreislaufreaktor bestand ebenfalls aus einem 8 L-Vorlagebehälter und einem 12 L-Rohrreaktor, der mit 10 kg 0,5% Pd/ZrO₂ Festbettkatalysator bei einer Temperatur von 200°C gefüllt war.

Aus dem zweiten Reaktionskreislauf kam eine Mischung von 73% CDON, 4% CDAN-Epoxid, 16% CDAN, 0,4% CDEN und 2% CDOL. Dieses Gemisch wurde in ein mit 2,7 kg Pd/ZrO₂ gefülltem Festbettreaktor mit einer Dosierrate von 2 kg/h zudosiert. In diesem Rohr wurde die Reaktionsmischung in Kontakt mit N₂ (4 L/h) und H₂ (16 L/h) bei einem Gesamtdruck von 1,7 bar und einer Temperatur von 205 °C gebracht. Aus diesem Rohr wurde eine Rohmischung aus 78% CDON, 17% CDAN und 2,2% CDOL erhalten. Die Komponenten dieses Gemisches wurden mittels Destillation voneinander getrennt und aufgereinigt.

### Destillation Leichtsiederfraktion:

Die Rohmischung aus der Umlagerung wurde in einem ersten kontinuierlichen Destillationschritt von einer Leichtsiederfraktion (Komponenten, die einen geringeren Siedepunkt als CDON besitzen) befreit. Die verwendete Kolonne war mit 8 m Gewebepackung mit einer Oberfläche von 500 m²/m³ ausgerüstet und wurde bei einem Kopfdruck von 10 mbar betrieben.

Die Verbindungen aus dieser Leichsiederfraktion, die weniger oder gleich 11 Kohlenstoffatome enthalten, wurden in einer zusätzlichen Destillationskolonne vom CDAN abgetrennt. Das erhaltene CDAN mit einer Reinheit von 99% wurde in die Oxidation mit Borsäure (konventionelle Route) geführt.

### Destillation von CDON:

Der Sumpf aus der ersten Destillation wurde in einem weiteren kontinuierlichen Destillationschritt (Kolonne mit 6 m Gewebepackung; Oberfläche von 500 m²/m³) destilliert und CDON bei einem Kopfdruck von 10 mbar als Destillat gewonnen. Komponenten mit einem höheren Siedepunkt als CDON (zum Beispiel CDOL) wurden als Sumpfprodukt abgetrennt. Die Reinheit des gewonnen CDON betrug > 99% und als CDON-Ausbeute wurden in der Destillation 98% erreicht.

Dieses Cyclododecanon konnte mit bekannten Verfahren weiter zu Laurinlactam umgesetzt werden.

Der Sumpf dieser zweiten Destillation wurde in einer zusätzlichen Kolonne weiter aufgereinigt: CDOL wurde in einer zusätzlichen Kolonne von anderen Hochsiedern abgetrennt und in die Dehydrierung (konventionelle Route) geführt.

### Verwertung vom anfallenden CDAN in die konventionelle Route:

Das CDAN (1 kg/h) aus der Epoxidroute wurde mit Borsäure (0,05 kg/h) umgesetzt. Bei einer Verweilzeit von 2 h Reaktion bei 155 °C und 132 l/h Sauerstoff-Zufuhr wurde ein CDAN-Umsatz von 8,5% und eine Selektivität bezüglich CDON und CDOL von 88% erhalten. Dieser Umsatz und die Zusammensetzung entsprachen dem Umsatz und der Selektivität aus der konventionellen Route. Die Mischung konnte analog zum konventionellen Produkt weiter zu Laurinlactam umgesetzt werden.

### Verwertung vom anfallenden CDOL in die konventionelle Route:

1 kg CDOL aus der Epoxidroute wurde mit 10 kg CDOL/CDON (70:30 w:w) Mischung der konventionellen Route in einem 30 L Dehydrierungsreaktor zusammengemischt. Der Reaktor war mit 1,9 kg Cu-haltigen Katalysator (H10167 von Evonik) gefüllt. Nach 6 h Reaktion bei 240 °C wurde ein Gemisch von CDON (10,5 kg) und CDOL (0,5 kg) erhalten. Das CDON wurde mit einer Reinheit von über 99,8% abdestilliert und das CDOL wurde in die Dehydrierung zurückgeführt. Die Qualität des erhaltenen CDON entsprach der Qualität des Cyclododecanons aus der konventionellen Route und konnte mit dieser Route weiter zu Laurinlactam umgesetzt werden.

### Beispiel 2

Das CDAN (oder die Mischung von CDAN und CDEN) kann alternativ verwertet werden. Statt das CDAN in die Oxidation mit Borsäure zu überführen, kann man es in der Vorstufe (Hydrierung von CDT in CDAN) verwenden. Es gilt im Besonderen, wenn das CDAN aus der Epoxidroute eine signifikante Menge an CDEN enthält.

### Verwertung der Mischung von CDAN und CDEN in der CDT Hydrierung:

Die Mischung von CDAN und CDEN (0,04 kg/h) aus der Epoxidroute wurde mit CDT (0,36 kg/h) in einem 0,4 L Hydrierungsreaktor zusammengemischt. Der Hydrierungsreaktor war mit 0,284 kg 0,5% Pd/Al₂O₃ gefüllt. Bei 160 °C und 15 bar Wasserstoff wurde CDAN mit einer Reinheit von 98,5% erhalten. Diese Reinheit entsprach der Reinheit des Cyclododecans in der konventionellen Route bei den entsprechenden Reaktionsbedingungen und konnte mit dieser Route weiter zu Laurinlactam umgesetzt werden.

## Patentansprüche

1. Verfahren zur Herstellung von Cyclododecanon (CDON) mittels einer Reaktionsroute I, umfassend die Schritte
a. Epoxidierung von Cyclododecen (CDEN) zu Epoxycyclododecan (CDANepoxid) und
b. Umlagerung des CDANepoxids zu CDON unter Erhalt eines Gemisches, das Cyclododecan (CDAN) enthält,
**dadurch gekennzeichnet, dass** CDAN aus dem CDON-haltigen Gemisch abgetrennt und zu CDON oxidiert wird und
das abgetrennte CDAN vor Durchführung der Oxidation einer Reaktionsroute II zur Herstellung von CDON zugeführt wird, welche umfasst
i. Hydrierung von Cyclododecatrien (CDT) zu CDAN,
ii. Oxidation von CDAN zu einem Gemisch umfassend Cyclododecanol (CDOL) und CDON und
iii. Dehydrierung von CDOL zu CDON.

2. Verfahren nach Anspruch 1, **dadurch gekennzeichnet, dass** der die Umlagerung (Schritt b) in Gegenwart eines edelmetallhaltigen Katalysators erfolgt.

3. Verfahren nach Anspruch 2, **dadurch gekennzeichnet, dass** der Katalysator der Umlagerung (Schritt b) Titaniumdioxid, Zirkoniumdioxid oder beides enthält.

4. Verfahren nach einem der vorherigen Ansprüche, **dadurch gekennzeichnet, dass** das CDANepoxid aus dem Schritt a CDAN enthält, das vor der Umlagerung (Schritt b) zumindest teilweise abgetrennt wird.

5. Verfahren nach Anspruch 4, **dadurch gekennzeichnet, dass** das vor der Umlagerung abgetrennte CDAN zu CDON oxidiert wird.

6. Verfahren nach einem der vorherigen Ansprüche, **dadurch gekennzeichnet, dass** CDON-haltige Gemisch CDOL enthält, das zu CDON dehydriert wird.

7. Verfahren nach Anspruch 6, **dadurch gekennzeichnet, dass** das CDOL vor der Dehydrierung aus dem CDON-haltigen Gemisch abgetrennt und der Reaktionsroute II zur Herstellung von CDON zugeführt wird

8. Verfahren nach einem der vorherigen Ansprüche, **dadurch gekennzeichnet, dass** das abgetrennte CDAN der Oxidation von CDAN gemäß Schritt b der Reaktionsroute II zugeführt wird.

9. Verfahren nach Anspruch 8, **dadurch gekennzeichnet, dass** das CDAN bis zu 0,5 Gew.-% CDEN, bezogen auf das Gesamtgewicht aus CDAN und CDEN, enthält.

10. Verfahren nach einem der vorherigen Ansprüche, **dadurch gekennzeichnet, dass** das abgetrennte CDAN der Hydrierung von CDT gemäß Schritt a der Reaktionsroute II zugeführt wird, wobei das CDAN 0,1 bis bis 99 Gew.-% CDEN, bezogen auf das Gesamtgewicht aus CDAN und CDEN, enthält.

11. Verfahren nach einem der vorherigen Ansprüche, **dadurch gekennzeichnet, dass** mindestens eine Abtrennung, vorzugsweise alle Abtrennungen, mittels Destillation erfolgen.

12. Verfahren zur Synthese von Laurinlactam aus CDON, umfassend die Herstellung von CDON nach einem Verfahren gemäß einem der vorherigen Ansprüche.

13. Verfahren nach Anspruch 12, **dadurch gekennzeichnet, dass** das CDON zu Cyclododecanon-oxim (CDON-oxim) umgesetzt wird.

14. Verfahren zur Herstellung von Polyamid 12 aus CDON, umfassend die Herstellung von CDON nach einem Verfahren gemäß einem der Ansprüche 1 bis 11.

## Claims

1. Process for preparing cyclododecanone (CDON) by means of a reaction route I, comprising the steps of
a. epoxidizing cyclododecene (CDEN) to epoxycyclododecane (CDAN epoxide) and
b. rearranging the CDAN epoxide to CDON to obtain a mixture comprising cyclododecane (CDAN),
**characterized in that** CDAN is separated from the CDON-containing mixture and oxidized to CDON and the CDAN separated, prior to performance of the oxidation, is sent to a reaction route II for preparation of CDON, comprising
i. hydrogenation of cyclododecatriene (CDT) to CDAN,
ii. oxidation of CDAN to give a mixture comprising cyclododecanol (CDOL) and CDON and
iii. dehydrogenation of CDOL to CDON.

2. Process according to Claim 1, **characterized in that** the rearrangement (step b) is effected in the presence of a noble metal catalyst.

3. Process according to Claim 2, **characterized in that** the catalyst for the rearrangement (step b) comprises titanium dioxide, zirconium dioxide or both.

4. Process according to any of the preceding claims, **characterized in that** the CDAN epoxide from step a comprises CDAN which is at least partly removed prior to the rearrangement (step b).

5. Process according to Claim 4, **characterized in that** the CDAN separated prior to the rearrangement is oxidized to CDON.

6. Process according to any of the preceding claims, **characterized in that** the CDON-containing mixture comprises CDOL which is dehydrogenated to CDON.

7. Process according to Claim 6, **characterized in that** the CDOL is separated from the CDON-containing mixture prior to the dehydrogenation and is sent to the reaction route II for preparation of CDON.

8. Process according to any of the preceding claims, **characterized in that** the CDAN separated is sent to the oxidation of CDAN as per step b of reaction route II.

9. Process according to Claim 8, **characterized in that** the CDAN contains up to 0.5% by weight of CDEN, based on the total weight of CDAN and CDEN.

10. Process according to any of the preceding claims, **characterized in that** the CDAN separated is sent to the hydrogenation of CDT as per step a of reaction route II, the CDAN containing 0.1% to 99% by weight of CDEN, based on the total weight of CDAN and CDEN.

11. Process according to any of the preceding claims, **characterized in that** at least one removal is conducted, preferably all the removals are conducted, by means of distillation.

12. Process for synthesizing laurolactam from CDON, comprising the preparation of CDON by a process according to any of the preceding claims.

13. Process according to Claim 12, **characterized in that** the CDON is converted to cyclododecanone oxime (CDON oxime).

14. Process for preparing polyamide 12 from CDON, comprising the preparation of CDON by a process according to any of Claims 1 to 11.

## Revendications

1. Procédé de fabrication de cyclododécanone (CDON) par une voie de réaction I, comprenant les étapes suivantes :
a. l'époxydation de cyclododécène (CDEN) en époxycyclododécane (CDAN-époxyde) et
b. le réarrangement du CDAN-époxyde en CDON pour obtenir un mélange qui contient du cyclododécane (CDAN), **caractérisé en ce que** le CDAN est séparé du mélange contenant de la CDON et oxydé en CDON, et
le CDAN séparé est introduit avant la réalisation de l'oxydation dans une voie de réaction II pour la fabrication de CDON, qui comprend :
i. l'hydrogénation de cyclododécatriène (CDT) en CDAN,
ii. l'oxydation du CDAN en un mélange comprenant du cyclododécanol (CDOL) et de la CDON, et
iii. la déshydrogénation du CDOL en CDON.

2. Procédé selon la revendication 1, **caractérisé en ce que** le réarrangement (étape b) a lieu en présence d'un catalyseur contenant des métaux nobles.

3. Procédé selon la revendication 2, **caractérisé en ce que** le catalyseur du réarrangement (étape b) contient du dioxyde de titane, du dioxyde de zirconium ou les deux.

4. Procédé selon l'une quelconque des revendications précédentes, **caractérisé en ce que** le CDAN-époxyde de l'étape a contient du CDAN, qui est au moins partiellement séparé avant le réarrangement (étape b).

5. Procédé selon la revendication 4, **caractérisé en ce que** le CDAN séparé avant le réarrangement est oxydé en CDON.

6. Procédé selon l'une quelconque des revendications précédentes, **caractérisé en ce que** le mélange contenant de la CDON contient du CDOL qui est déshydrogéné en CDON.

7. Procédé selon la revendication 6, **caractérisé en ce que** le CDOL est séparé du mélange contenant de la CDON avant la déshydrogénation et introduit dans la voie de réaction II pour la fabrication de CDON.

8. Procédé selon l'une quelconque des revendications précédentes, **caractérisé en ce que** le CDAN séparé est introduit dans l'oxydation de CDAN selon l'étape b de la voie de réaction II.

9. Procédé selon la revendication 8, **caractérisé en ce que** le CDAN contient jusqu'à 0,5 % en poids de CDEN, par rapport au poids total de CDAN et de CDEN.

10. Procédé selon l'une quelconque des revendications précédentes, **caractérisé en ce que** le CDAN séparé est introduit dans l'hydrogénation de CDT selon l'étape a de la voie de réaction II, le CDAN contenant 0,1 à 99 % en poids de CDEN, par rapport au poids total de CDAN et CDEN.

11. Procédé selon l'une quelconque des revendications précédentes, **caractérisé en ce qu'**au moins une séparation, de préférence toutes les séparations, ont lieu par distillation.

12. Procédé de synthèse de laurine-lactame à partir de CDON, comprenant la fabrication de CDON par un procédé selon l'une quelconque des revendications précédentes.

13. Procédé selon la revendication 12, **caractérisé en ce que** la CDON est transformée en cyclododécanone-oxime (CDON-oxime).

14. Procédé de fabrication de polyamide 12 à partir de CDON, comprenant la fabrication de CDON par un procédé selon l'une quelconque des revendications 1 à 11.
